# EUROPÄISCHE PATENTSCHRIFT

(11) **EP 0 966 455 B1**
(45) Veröffentlichungstag und Bekanntmachung des Hinweises auf die Patenterteilung: **28.01.2004**
(21) Anmeldenummer: 98909391.9
(22) Anmeldetag: 05.02.1998
(51) Int. Cl.: C07D 303/48, A61K 31/335

(54) **OXIRANCARBONSÄUREN FÜR DIE BEHANDLUNG VON DIABETES**
OXIRAN CARBOXYLIC ACIDS FOR THE TREATMENT OF DIABETES
ACIDES CARBOXYLIQUES D'OXIRANE S'UTILISANT DANS LE TRAITEMENT DU DIABETE

(30) Priorität: 14.02.1997 DE 19705718
(43) Veröffentlichungstag der Anmeldung: 29.12.1999
(73) Patentinhaber: Wolf, Horst P.O., Dr., 78476 Allensbach (DE)
(72) Erfinder: Wolf, Horst P.O., Dr., 78476 Allensbach (DE)
(74) Vertreter: Benz, Jürgen, Dr.
(86) Internationale Anmeldenummer: PCT/EP1998/000611
(87) Internationale Veröffentlichungsnummer: WO 1998/035952

(56) Entgegenhaltungen:
- WO-A-83/00334
- US-A- 4 324 796
- US-A- 4 337 267
- US-A- 4 788 306

## Beschreibung

### Anwendungsgebiet der Erfindung

Die Erfindung betrifft neue Arylalkyl- bzw. Aryloxyalkyl-substituierte Oxirancarbonsäuren, Verfähren zu ihrer Herstellung, ihre Anwendung und sie enthaltende Arzneimittel.

### Stand der Technik

In der EP 0 046 590 werden hypoglykämisch und hypoketonämisch wirksame Phen(alk)oxy-substituierte Oxirancarbonsäuren und deren Ester der allgemeinen Formel A beschrieben 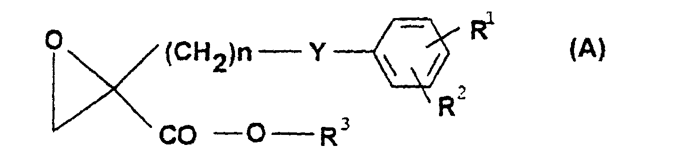 worin
- R¹: ein Wasserstoffatom, ein Halogenatom, eine 1-4 C-Niederalkylgruppe, eine 1-4 C-Niederalkoxygruppe, eine Nitrogruppe oder eine Trifluormethylgruppe bedeutet,
- R²: eine Bedeutung von R1 hat,
- R³: ein Wasserstoffatom oder eine 1-4 C-Niederalkylgruppe,
- Y: die Gruppierung -O-(CH₂)ₘ -,
- m: 0 oder eine ganze Zahl von 1 bis 4 und
- n: eine ganze Zahl von 1 bis 8 bedeuten,
wobei die Summe von m und n eine ganze Zahl von 2 bis 8 ist, sowie die Salze der Carbonsäuren.

In der EP 0 231 367 B1 ist die Verwendung der Verbindungen der allgemeinen Formel A für die Verhütung und/oder Behandlung von Krankheiten, die auf einer erhöhten Cholesterin- und/oder Triglycerid-Konzentration im Organismus beruhen, beschrieben.

In der DE-OS 4 340 879 A1 ist der Einsatz der Verbindungen der allgemeinen Formel A bei der Verhütung und/oder Behandlung der Herzinsuffizienz beschrieben.

In der DE-OS 3 032 668 werden u.a. nicht-aromatische Cycloalkyl(alk)oxysubstituierte Oxirancarbonsäuren beschrieben.

In der EP 0 283 168 werden Phenylalkyl- und Phenoxyalkyloxirancarbonsäuren und deren Ester mit 1-2 Fluorsubstituenten in der Alkylkette beschrieben, die als Fettsäureoxidationsinhibitoren mit geringem Schädigungspotential für die Herzmuskelfunktion wirken sollen.

US 4,324,796 beschreibt 2-Arylalkyl-substituierte Oxiran-2-carbonsäurederivate, wobei der Arylrest zwei Substituenten R¹ und R² aufweist, und R¹ und R² ein Wasserstoff, Halogen, eine Hydroxylgruppe, eine Niederalkyl-, Niederalkoxy- oder eine Trifluormethylgruppe sein kann. Diese Verbindungen weisen hypoglykämische Wirkung auf.

US 4,337,267 beschreibt 2-Aryloxyalkyl-substituierte Oxiran-2-carbonsäurederivate, insbesondere 2-(6-(4-Chlorphenoxy)hexyl)oxiran-2-carbonsäureethylester, wobei der Arylrest Substituenten R¹ und R² aufweist, und R¹ und R² ein Wasserstoff, Halogen, eine Niederalkyl-, Niederalkoxy-, eine Nitro- oder eine Trifluormethylgruppe sein kann. Diese Verbindungen weisen hypoglykämische Wirkung auf.

US 4,788,306 beschreibt Verbindungen der allgemeinen Formel 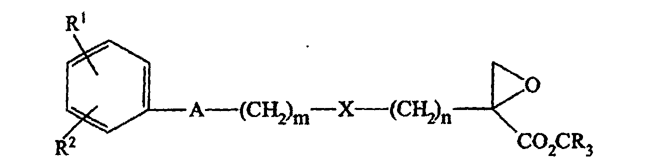 wobei A die Gruppe -O- sein kann, sowie R¹ und R² unabhängig voneinander Wasserstoff, eine Hydroxylgruppe, eine halogenierte oder nicht-halogenierte Niederalkylgruppe, eine Niederalkoxygruppe, eine halogenierte Niederalkylsulfonylgruppe, ein Halogen oder eine Nitrogruppe sein können. X ist ausgewählt aus der Gruppe bestehend aus -CH₂-CH(F)-, -CH₂-CF₂- oder -CH=CF-. Diese Verbindungen werden bezüglich ihrer Eignung zur Behandlung von Diabetes untersucht.

WO 83/00334 beschreibt Verbindungen der allgemeinen Formel 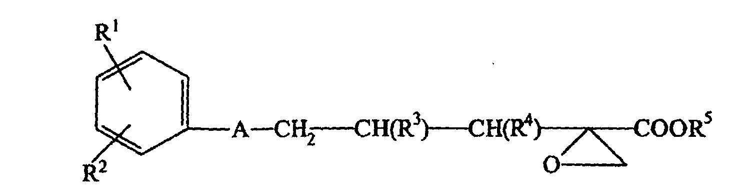 wobei R¹ und R² Wasserstoff, ein Halogenatom, eine Niederalkylgruppe, eine Niederalkoxygruppe oder eine Trifluormethylgruppe sein kann. Die Verbindungen werden bezüglich ihrer Eignung zur Behandlung von Diabetes untersucht.

### Beschreibung der Erfindung

Gegenstand der Erfindung sind neue Arylalkyl- bzw. Aryloxyalkyl-substituierte Oxirancarbonsäuren der allgemeinen Formel I 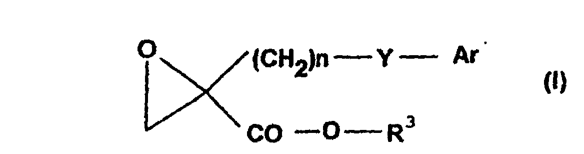 worin
- Ar: einen substituierten Phenylrest 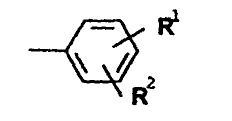 einen mit einem Rest R⁴ substituierten 1- oder 2-Naphthylrest oder einen heterocyclischen Rest Het,
- R¹: ein Wasserstoffatom, ein Halogenatom oder eine 1-4 C-Niederalkylgruppe,
- R²: eine der Gruppen 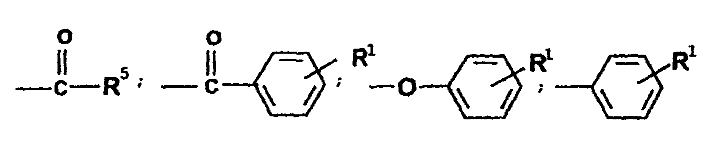 oder eine vollständig oder überwiegend mit Fluor substituierte 1-3 C-Alkoxygruppe,
- R³: ein Wasserstoffatom oder eine 1-4 C-Niederalkylgruppe,
- R⁴: ein Wasserstoffatom, eine 1-4 C-Niederalkylgruppe, eine gegebenfalls vollständig oder überwiegend mit Fluor substituierte 1-3 C-Alkoxygruppe oder ein Halogenatom,
- R⁵: eine 1-4 C-Niederalkylgruppe,
- Y: die Gruppierung - O - ,
- n: eine ganze Zahl von 2-8 und
- Het: einen heterocyclischen Ring mit 5 Gliedern und aus der Gruppe Thiophen, Thiazol, Isothiazol, Pyrrol und besonders bevorzugt Pyrazol, welcher 1-2 gleiche oder verschiedene Substituenten R' tragen kann,
bedeuten, wobei die Kette - (CH₂)ₙ - gegebenfalls auch durch ein Glied - CH(CH₃) - oder - C(CH₃)₂ - unterbrochen sein kann, sowie die Salze der entsprechenden Carbonsäuren (R³ = H).

Die 1-4 C-Niederalkylreste können geradkettig oder verzweigt sein. Geradkettige Alkylreste sind beispielsweise der Methyl-, Ethyl-, n-Propyl- und der Butylrest, von denen die mit 1 bis 2 Kohlenstoffatomen bevorzugt sind. Verzweigte Alkylreste sind beispielsweise der Isopropyl-, Isobutyl und der sek.-Butylrest, von denen der mit 3 Kohlenstoffatomen bevorzugt ist. Als Alkylreste von Niederalkoxygruppen kommen sowohl geradkettige als auch verzweigte Niederalkylgruppen in Frage. Die Methoxy-gruppe ist als Niederalkylgruppe bevorzugt. Als Alkylreste in Acylgruppen kommen sowohl geradkettige als auch verzweigte Niederalkylgruppen in Frage, von denen die Methylgruppe und die tert. Butylgruppe bevorzugt sind.

Halogenatome sind Fluor-, Chlor- und Bromatome, von denen Fluor, insbesondere Chlor bevorzugt ist.

In den substituierten Phenylresten Ar stehen die Substituenten R¹ und R² bevorzugt in m- oder p-Stellung und R¹ ist bevorzugt ein Wasserstoffatom.

Von den vollständig oder überwiegend mit Fluor substituierten 1-3 C-Alkoxygruppen sind die Trifluor-methoxy-, die 2,2,2-Trifluorethoxy-, die 1,1,2,2-Tetrafluorethoxygruppe und insbesondere die Difluormethoxygruppe bevorzugt.

Als Salze kommen Salze mit anorganischen und organischen Basen in Betracht. Pharmakologisch nicht verträgliche Salze werden nach an sich bekannten Methoden in pharmakologisch, d.h. biologisch verträgliche Salze überführt, die unter den erfindungsgemäßen Salzen bevorzugt sind. Als Kationen für die Salzbildung werden vor allem die Kationen der Alkalimetalle, Erdalkalimetalle oder Edelmetalle verwendet, es kommen jedoch auch die entsprechenden Kationen organischer Stickstoffbasen, wie Amine, Aminoalkohole, Aminozucker, basische Aminosäuren etc. zur Anwendung.

Beispielsweise seien Salze von Lithium, Natrium, Kalium, Magnesium, Calcium, Aluminium, Ethylendiamin, Dimethylamin, Diethylamin, Morpholin, Piperidin, Piperazin, N-Niederalkylpiperazin (z.B. N-Methylpiperazin), Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methyl-glucamin, Glucosamin, N-Methylglucosamin, Lysin, Ornithin, Arginin, Chinolin genannt.

Die erfindungsgemäßen Arylalkyl- bzw. Aryloxyalkyloxirancarbonsäuren der allgemeinen Formel I besitzen ein Chiralitätszentrum. Die Erfindung schließt daher sowohl die Racemate und die Enantiomeren als auch deren Gemische ein. Für die Racemattrennung der Carbonsäuren sind Salze mit optisch aktiven Basen wie Cinchonidin oder Dehydroabietylamin besonders bevorzugt.

Die erfindungsgemäßen Verbindungen weisen wertvolle pharmakologische Eigenschaften auf, die sie gewerblich verwertbar machen. Sie wirken hypoglycämisch und lipidsenkend und verbessern die Wirksamkeit von Insulin bei der Behandlung von insulinresistenten Zuständen, wie z.B. beim Metabolischen Syndrom und insbesondere beim Diabetes Typ 2.

Sie sind den bekannten Oxirancarbonsäuren des Standes der Technik in folgender Weise überlegen:
a) Sie zeichnen sich durch einen unter bestimmten Bedingungen deutlich besseren
   therapeutischen Index in der Weise aus, daß die bei einzelnen Typ 2-Diabetikern auftretenden Anstiege der Leberenzyme (Transaminasen) gar nicht oder in deutlich geringerem Ausmaß auftreten,
b) sie zeigen eine überlegene Wirkung hinsichtlich der Steigerung der Insulinwirkung bei insulinresistenten Zuständen
c) sie werden schneller metabolisiert und bilden keine langlebigen Metaboliten.

Aufgrund ihrer vorteilhaften und überlegenen Wirksamkeit sind die erfindungsgemäßen Verbindungen der allgemeinen Formel I sowie die pharmakologisch verträglichen Salze zur human- und veterinärmedizinischen Behandlung und Prophylaxe von Krankheiten, die auf Störungen des Glucose- und Fettstoffwechsels beruhen, geeignet.

Beispielsweise werden sie eingesetzt zur Behandlung prädiabetischer Zustände; zur Behandlung und Verhinderung der Manifestation des Diabetes Typ 2 sowie aller krankhaften Zustände, die mit einer pathologischen Insulinresistenz einhergehen; zur Behandlung und Verhinderung der Manifestation aller krankhaften Zustände mit pathologisch erhöhter Ketonkörperproduktion; zur Behandlung und Verhinderung der Manifestation aller krankhaften Zustände, die auf erhöhten Cholesterinund/oder Triglycerid-Konzentrationen im Blut beruhen (Hyperlipidämie, Arteriosklerose, koronare Herzerkrankung).

Gegenstand der Erfindung sind auch die erfindungsgemäßen Verbindungen zur Anwendung bei der Behandlung und Prophylaxe der angegebenen Krankheiten.

Ein weiterer Gegenstand der Erfindung sind Arzneimittel, die eine oder mehrere der Arylalkyl- bzw. Aryloxyalkyloxirancarbonsäuren der allgemeinen Formel I 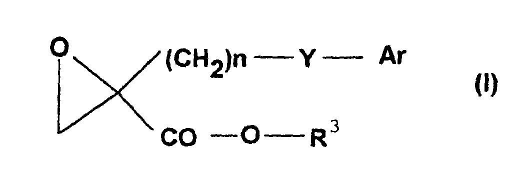 worin
- Ar: einen substituierten Phenylrest 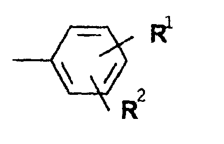 einen mit einem Rest R⁴ substituierten 1- oder 2-Naphthylrest oder einen heterocyclischen Rest Het,
- R¹: ein Wasserstoffatom, ein Halogenatom oder eine 1-4 C-Niederalkylgruppe,
- R²: eine der Gruppen 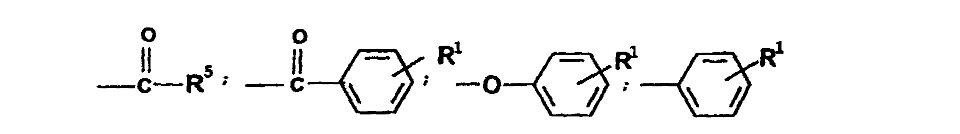 oder eine vollständig oder überwiegend mit Fluor substituierte 1-3 C-Alkoxygruppe,
- R³: ein Wasserstoffatom oder eine 1-4 C-Niederalkylgruppe,
- R⁴: ein Wasserstoffatom, eine 1-4 C-Niederalkylgruppe, eine gegebenfalls vollständig oder überwiegend mit Fluor substituierte 1-3 C-Alkoxygruppe oder ein Halogenatom,
- R⁵: eine 1-4 C-Niederalkylgruppe,
- Y: die Gruppierung - O -,
- n: eine ganze Zahl von 2-8 und
- Het: einen heterocyclischen Ring mit 5 Gliedern und aus der Gruppe Thiophen, Thiazol, Isothiazol, Pyrrol und besonders bevorzugt Pyrazol, welcher 1-2 gleiche oder verschiedene Substituenten R¹ tragen kann,
bedeuten, wobei die Kette - (CH₂)ₙ - gegebenfalls auch durch ein Glied - CH(CH₃)-oder - C(CH₃)₂ - unterbrochen sein kann, sowie die pharmakologisch verträglichen Salze der Carbonsäuren (R³ = H) mit anorganischen oder organischen Basen enthalten.

Gegenstand der Erfindung ist außerdem die Verwendung der erfindungsgemäßen Verbindungen zur Herstellung von Arzneimitteln zur Bekämpfung der angegebenen Krankheiten.

Die Arzneimittel werden nach an sich bekannten Verfahren hergestellt. Als Arzneimittel werden die erfindungsgemäßen Verbindungen entweder als solche oder gegebenenfalls in Kombination mit geeigneten pharmazeutischen Trägerstoffen eingesetzt. Enthalten die pharmazeutischen Zubereitungen neben den Wirkstoffen pharmazeutische Trägerstoffe, beträgt der Wirkstoffgehalt dieser Mischung 1 bis 95, vorzugsweise 10 bis 85 % (w/w) der Gesamtmischung. Die Arzneimittel werden beispielsweise für die orale oder parenterale (intravenöse, intramuskuläre) Gabe in geeigneten Dosen formuliert. Die Tagesdosis für die orale Applikation am Menschen liegt im allgemeinen zwischen 0.1 und 30, vorzugsweise 0.3 und 15, insbesondere 0,6 und 3 mg / kg Körpergewicht. Die Dosierung für die parenterale Behandlung liegt zwischen 0.3 und 1 mg Wirkstoff / kg Körpergewicht.
Die pharmazeutischen Zubereitungen bestehen bevorzugt aus den erfindungsgemäßen Wirkstoffen und nicht-toxischen, pharmazeutisch verträglichen Arzneimittelträgern, die als Zumischung oder Verdünnungsmittel in fester, halbfester, oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Neben den erfindungsgemäßen Verbindungen der allgemeinen Formel I, in denen die Substituenten die oben angegebene Bedeutung haben, und/oder ihren Salzen können die pharmazeutischen Zubereitungen weiterhin einen oder mehrere pharmakologisch aktive Bestandteile anderer Arzneimittelgruppen, wie Antidiabetika (Sulfonamide, Sulfonylharnstoffe, Thiazolidindione u.a.) oder Hypolipidämika (Nikotinsäure und deren Derivate, Clofibrate, HMG-CoA-Reduktasehemmer) enthalten.

Die erfindungsgemäßen Verbindungen werden nach an sich bekannten Verfahren hergestellt. Ausführliche Anweisungen für die Herstellung der prinzipiellen Verbindungsklasse sind in der eingangs genannten EP 0046 590 beschrieben, welche in bezug auf die Verfahrenstechnik in die Offenbarung dieser Anmeldung mit eingeschlossen ist. Diese Vorschriften können in Analogieverfahrensschritten auf die neuen erfindungsgemäßen Verbindungen angewendet werden. Die Einführung der neuen erfindungsgemäßen, chemisch aber an sich übliche Bedeutungen für den Rest R² im Vergleich zu der besagten europäischen Anmeldung kann der Fachmann ohne weiteres nach zahlreichen Standardmethoden bewerkstelligen.

Die Verbindungen der allgemeinen Formel I fallen dabei normalerweise in Form von racemischen Gemischen an, die mittels bekannter Verfahren in die Enantiomeren getrennt werden. Beispielsweise wandelt man mit einem optisch aktiven Spaltungsmittel das Racemat in Diastereomere um, die anschließend durch selektive Kristallisation getrennt und in die entsprechenden optischen Isomeren überführt werden. Als optisch aktive Spaltungsmittel dienen z.B. optisch aktive Basen, wie 1- und d-1-Phenyl-ethylamin, Cinchonidin oder d-Ephedrin, aus denen Salze der Säuren der allgemeinen Formel I, oder optisch aktive Alkohole, wie Borneol oder Menthol, mit denen Ester aus den Säuren der allgemeinen Formel I hergestellt werden. Als besonders geeignet hat sich die Racematspaltung der Säuren mittels Dehydroabiethylamin als Salzbildner herausgestellt.

Die folgenden Beispiele sollen die Erfindung näher erläutern, ohne sie danei einzuschränken.

### Beispiel 1:

Die erfindungsgemäßen Verbindungen der Formel I erniedrigen die Glucosekonzentration im Blut von Ratten, die durch längeres Fasten in einen insulinresistenten Zustand gebracht worden sind. Sie zeigen sich bei dieser Wirkung den aus dem Stand der Technik bekannten Wirkstoffen, z.B. rac-Etomoxir (siehe EP 046 590) überlegen.

In der folgenden Tabelle werden die untersuchten repräsentativen Substanzen zur Kennzeichnung nummeriert:

| Nummer | Name der Verbindung |
|---|---|
| **1** | 2-(6-(4-Chlorphenoxy)hexyl)oxiran-2-carbonsäureethylester (Vergleichsverbindung) |
| **2** | 2-(6-(4-Difluormethoxyphenoxy)hexyl)oxiran-2-carbonsäure-ethylester |
| **3** | 2-(5-(4-Difluormethoxyphenoxy)pentyl)oxiran-2-carbonsäure-ethylester |
| **4** | 2-(5-(4-Acetylphenoxy)pentyl)oxiran-2-carbonsäureethylester |

In der Tabelle 1 sind folgende Befunde dargestellt:
- *In Spalte A*: die blutglucosesenkende Wirkung der repräsentativen Substanzen an insulinresistenten Ratten (nach 24 Stunden Fasten) 2 Stunden nach oraler Administration von äquimolaren Dosen (100 umol/kg Körpergewicht),
- *in Spalte B*: die triglyceridsenkende Wirkung der repräsentativen Substanzen im Blutplasma von gefütterten, gesunden Ratten nach 16-tägiger oraler Administration äquimolare Dosen (100 umol/kg Körpergewicht) 24 Stunden nach der letzten Substanzadministration,
- *in Spalte C*: die cholesterinsenkende Wirkung der repräsentativen Substanzen im Blutplasma von gefütterten, gesunden Ratten nach 16-tägiger oraler Administration von äquimolaren Dosen (100 umol/kg Körpergewicht) 24 Stunden nach der letzten Substanzadministration.
Angegeben werden prozentuale Änderungen der mit Substanz behandelten Tiere im Vergleich zu mit Placebo behandelten Kontrolltieren, errechnet aus den Mittelwerten von jeweils 10 Einzelwerten.

**Tabelle 1**

| **Substanznummer** | **(A) Glucose (%)** | **(B) Triglyceride (%)** | **(C) Cholesterin (%)** |
|---|---|---|---|
| **1** | **-18** | **-50** | **-15** |
| **2** | **-27** | **-69** | **-13** |
| **3** | **-27** | **-73** | **-26** |
| **4** | **-7** | **-41** | **-8** |

Die Überlegenheit der erfindungsgemäßen Verbindungen gegenüber dem Stand der Technik bezüglich der Glucosesenkung im Hungerzustand, gewählt als experimentelles Modell der Insulinresistenz, zeigt sich besonders am Beispiel der Substanzen Nr. 2 und 3 in Tab. 1. Unter Berücksichtigung der triglycerid- und cholesterinsenkenden Wirkung zeichnet sich besonders Substanz Nr. 3 durch eine dem Stand der Technik überlegene Wirkung aus.

### Beispiel 2:

In Tabelle 2 ist der Einfluß der repräsentativen Substanzen auf unerwünschte Nebeneffekte, die in der wissenschaftlichen Literatur beschrieben worden sind (K. Ratheiser, B. Schneeweiss et al.: Metabolism Clin. Exp. 40 (1991) 1185; H.P.O.Wolf in C.J. Bailey & P.R. Flatt, New antidiabetic drugs, Smith-Gordon, London 1990), dargestellt:
- *In Spalte A*: der transiente Anstieg der Aktivität des Leberenzyms Glutamat-Pyruvat-Transaminase (GPT) im Blutplasma nach 16-tägiger oraler Administration äquimolarer Dosen der Substanzen an gesunde, gefütterte Ratten 24 Stunden nach der letzten Substanzadministration,
- *in Spalte B*: der Anstieg des relativen Herzgewichtes (Herzgewicht / 100 g Körpergewicht) als Zeichen einer Herzhypertrophie nach 16-tägiger oraler Administration äquimolarer Dosen der Substanzen an gesunde, gefütterte Ratten 24 Stunden nach der letzten Substanzadministration,
- *in Spalte C*: der sicherheitspharmakologische Index, gebildet aus der prozentualen Senkung der Blutkonzentrationen Glucose + Triglyceride + Cholesterin dividiert durch die prozentualen Erhöhungen GPT-Aktivität + rel. Herzgewicht .

**Tabelle 2**

| **Substanznummer** | **(A) GPT-Aktivität (%)** | **(B) rel. Herzgew. (%)** | **(C) Sicherheitsindex (%)** |
|---|---|---|---|
| **1** | **8** | **14** | **3.77** |
| **2** | **5** | **14** | **5.74** |
| **3** | **12** | **11** | **5.48** |
| **4** | **-9** | **-2** | **56** |

Je höher der Index, desto größer ist die Sicherheit der Substanz zu beurteilen. Unter diesem Aspekt zeichnet sich die Substanz Nr. 4 als dem Stand der Technik besonders überlegen aus. Auch die Substanzen Nr. 2 und 3 sind dem Stand der Technik überlegen.

### Beispiel 3:

### Versuchstiere

Als Versuchstiere wurden männliche Sprague-Dawley Ratten der SPF-Zuchr Ivanovas (Kisslegg, Deutschland) mit einer Körpermasse von 255 - 400 g verwendet. Die Haltung der Tiere erfolgte konventionell zu je 4 Tieren in Mkrolonkäfigen (22 x 38 cm) in einem temperierten Raum (21 -23 Grad Celcius) mit festem Tag /Nacht-Rhythmus
(7/19 Uhr) und regulierter rel.Luftfeuchtigkeit von 55 - 60 %. Den Tieren wurde eine Haltungsdiät Altromin 1320 der Fa. Altromin (Lage,Deutschland) und Wasser ad libitum angeboten.

Zur Bestimmung der Substanzwirkung auf die Blutglucose wurde zur Erzeugung eines insulinresistenten Zustandes das Futter 24 Stunden vor der Substanzadministration entzogen.

Die Tiere wurden randomisiert in 5 Gruppen zu je 10 Tieren eingeteilt und markiert. Die Substanzen wurden den Tieren in Form einer neutralen, wäßrigen Emulsion (1 Gew.-Teil Substanz + 2 Gew.Teile Cremophor EL - ein Emulgator der Fa. BASF AG, Deutschland - ) mittels einer Magensonde in einem Volumen von 10 ml / kg Körper-gewicht verabreicht.

### Beispiel 4:

### Gewinnung von Blut und Serum

Zur Bestimmung der Glucose im Blut wurde den 24 Stunden nüchterenen Tieren 2 Stunden nach Substanz-administration 50 ul Blut mittels einer Galskapillare aus dem retrobulbären Venenplexus entnommen und in eiskalter Perchlorsäure (0,66 mol/l) enteiweißt. Nach Zentrifugation erfolgte die Bestimmung der Glucose im Überstand nach enzymatischen Standardverfahren.

Zur Bestimmung der Parameter Triglyceride, Cholesterin und der Aktivität der Glutamat-Pyruvat-Transaminase (GPT) wurde Blutplasma verwendet. Das Blutplasma wurde 15 Minuten nach der venösen Blutentnahme in heparinisierte Eppendorf- Reak-tionsgefäße durch Zentrifugation (2 x 2 Minuten bei 16 000 U/min in einer Eppendorf - Zentrifuge) als erythrocytenfreier Überstand gewonnen.

### Beispiel 5:

### Analytische Methoden

- *Glucose:*: Enzymatischer Test mit Hexokinase / Glucose-6-phosphatase, Testcombination von Boehringer Mannheim, Deutschland.
- *Triglyceride:*: Enzymatischer Test mit Lipase / Glycerokinase, Testkombination von Boehringer Mannheim, Deutschland.
- *Cholesterin:*: Enzymatischer Farbtest (CHOD-PAP-Methode), Testkombination von Boehringer Mannheim, Deutschland
- *GPT*:: Kinetischer Enzymtest, Testkombination von Boehringer Mannheim, Deutschland.
- *rel. Herzgewicht:*: Nach Tötung der Tiere durch Dekapitation und Entbluten durch Wägung festgestelltes Gewicht des vom rechten Vorhof befreiten Herzmuskels und Bezug auf 100 g Körpergewicht.

## Patentansprüche

1. Arylalkyl- bzw. Aryloxyalkyl-substituierte Oxirancarbonsäuren der allgemeinen Formel I 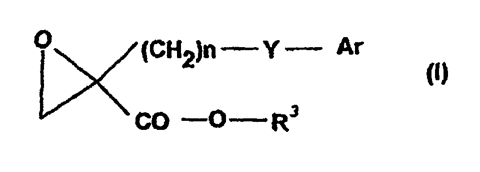 worin
Ar einen substituierten Phenylrest 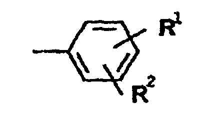 einen mit einem Rest R⁴ substituierten 1- oder 2-Naphthylrest oder einen heterocyclischen Rest Het,
R¹ ein Wasserstoffatom, ein Halogenatom oder eine 1-4 C-Niederalkylgruppe,
R² eine der Gruppen 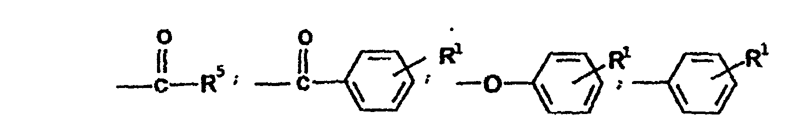 oder eine vollständig oder überwiegend mit Fluor substituierte 1-3 C- Alkoxygmppe,
R³ ein Wasserstoffatom oder eine 1-4 C-Niederalkylgruppe,
R⁴ ein Wasserstoffatom, eine 1-4 C-Niederalkylgruppe, eine gegebenfalls vollständig oder überwiegend mit Fluor substituierte 1-3 C-Alkoxygruppe oder ein Halogenatom,
R⁵ eine 1-4 C-Niederalkylgruppe,
Y die Gruppierung - O -
n eine ganze Zahl von 2-8 und
Het einen heterocyclischen Ring, mit 5 Gliedern und aus der Gruppe Thiophen, Thiazol, Isothiazol, Pyrrol und besonders bevorzugt Pyrazol, welcher 1-2 gleiche oder verschiedene Substituenten R¹ tragen kann,
bedeuten, wobei die Kette - (CH₂)ₙ - gegebenfalls auch durch ein Glied - CH(CH₃) - oder - C(CH₃)₂ - unterbrochen sein kann, sowie die Salze der Carbonsäuren (R³ = H).

2. Verbindung der Formel I gemäß Anspruch 1, ausgewählt aus der folgenden Gruppe:
2-(6-(4-Difluormethoxyphenoxy)hexyl)oxiran-2-carbonsäureethylester
2-(5-(4-Difluormethoxyphenoxy)pentyl)oxiran-2-carbonsäureethylester
2-(5-(4-Acetylphenoxy)pentyl)oxiran-2-carbonsäureethylester

3. Arzneimittel der Formel I nach Anspruch 1 oder 2.

4. Arzneimittel nach Anspruch 3 zur Anwendung bei der Behandlung und zur Prophylaxe von Krankheiten, die auf Störungen des Glucose- und/oder Fettstoffwechsels, wie
Zustände mit pathologischer Glucosetoleranz, Prädiabetes, Diabetes Typ 2, Zustände mit Insulinresistenz, Zustände mit pathologisch erhöhter Ketonkörperproduktion, Hyperlipidämie, Arteriosklerose und/oder koronarer Herzerkrankung, beruhen.

5. Pharmazeutischen Zubereitung, **dadurch gekennzeichnet, daß** sie eine oder mehrere Verbindungen der Formel I gemäß Anspruch 1 oder 2 gegebenenfalls zusammen mit Träger- und / oder Hilfsstoffen enthält.

6. Verwendung von Arylalkyl- bzw. Aryloxyalkyl-substituierte Oxirancarbonsäuren gemäß Anspruch 1 oder 2 zur Herstellung von Arzneimitteln für die Behandlung und zur Prophylaxe von Krankheiten, die auf Störungen des Glucoseund/oder Fettstoffwechsels, wie
Zustände mit pathologischer Glucosetoleranz, Prädiabetes, Diabetes Typ 2, Zustände mit Insulinresistenz,
Zustände mit pathologisch erhöhter Ketonkörperproduktion, Hyperlipidämie, Arteriosklerose und/oder koronarer Herzer-krankung, beruhen..

## Claims

1. Arylalkyl- or aryloxyalkyl-substituted oxiranecarboxylic acids of the general formula I 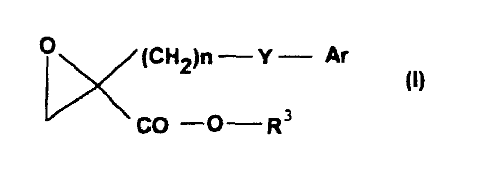 in which
Ar denotes a substituted phenyl radical 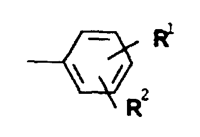 a 1- or 2-naphthyl radical which is substituted by an R⁴ radical, or a heterocyclic radical Het,
R¹ denotes a hydrogen atom, a halogen atom or a 1-4 C-(lower alkyl) group,
R² denotes one of the groups 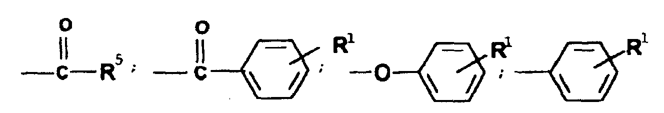 or a fully or predominantly fluorine-substituted 1-3 C-alkoxy group,
R³ denotes a hydrogen atom or a 1-4 C-(lower alkyl) group,
R⁴ denotes a hydrogen atom, a 1-4 C-(lower alkyl) group, an optionally fully or predominantly fluorine-substituted 1-3 C-alkoxy group or a halogen atom,
R⁵ denotes a 1-4 C-(lower alkyl) group,
Y denotes the -O- group,
n denotes an integer from 2 to 8, and
Het denotes a heterocyclic ring having 5 members from the group consisting of thiophene, thiazole, isothiazole, pyrrole and particularly preferably pyrazole, which may carry 1-2 identical or different substituents R¹,
where the -(CH₂)ₙ- chain may optionally also be interrupted by a -CH(CH₃)- or -C(CH₃)₂- member, and the salts of the carboxylic acids (R³ = H).

2. Compound of the formula I according to Claim 1, selected from the following group:
ethyl 2-(6-(4-difluoromethoxyphenoxy)hexyl)oxirane-2-carboxylate
ethyl 2-(5-(4-difluoromethoxyphenoxy)pentyl)oxirane-2-carboxylate
ethyl 2-(5-(4-acetylphenoxy)pentyl)oxirane-2-carboxylate.

3. Medicament of the formula I according to Claim 1 or 2.

4. Medicament according to Claim 3 for use in the treatment and for the prophylaxis of diseases based on disorders of glucose and/or fat metabolism, such as conditions with pathological glucose tolerance, prediabetes, type 2 diabetes, conditions with insulin resistance, conditions with pathologically increased ketogenesis, hyperlipidaemia, arteriosclerosis and/or coronary heart disease.

5. Pharmaceutical preparation, **characterized in that** it comprises one or more compounds of the formula I according to Claim 1 or 2, optionally together with excipients and/or adjuvants.

6. Use of arylalkyl- or aryloxyalkyl-substituted oxiranecarboxylic acids according to Claim 1 or 2 for the preparation of medicaments for the treatment and prophylaxis of diseases based on glucose and/or fat metabolism disorders, such as conditions with pathological glucose tolerance, prediabetes, type 2 diabetes, conditions with insulin resistance, conditions with pathologically increased ketogenesis, hyperlipidaemia, arteriosclerosis and/or coronary heart disease.

## Revendications

1. Acides oxiranecarboxyliques arylalkyl- ou aryloxyalkyl-substitués de formule générale I 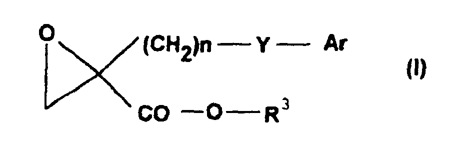 dans laquelle
Ar désigne un radical phényle substitué 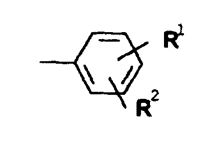 un radical 1- ou 2-naphtyle qui est substitué par un radical R⁴, ou un radical hétérocyclique Hét,
R¹ désigne un atome d'hydrogène, un atome d'halogène ou un groupement alkyle inférieur en C1-4,
R² désigne l'un des groupements 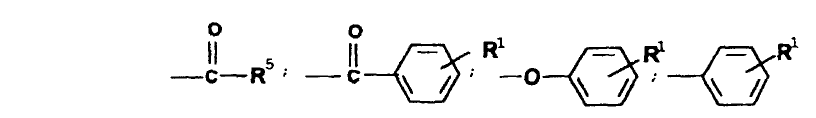 ou un groupement alkoxy en C1-3 substitué totalement ou en prédominance par fluor,
R³ désigne un atome d'hydrogène ou un groupement alkyle inférieur en C1-4,
R⁴ désigne un atome d'hydrogène, un groupement alkyle inférieur en C1-4, un groupement alkoxy en C1-3 éventuellement substitué totalement ou en pré-dominance par fluor, ou un atome d'halogène,
R⁵ désigne un groupement alkyle inférieur en C1-4,
Y désigne le groupement -O-,
n désigne un nombre entier allant de 2 à 8, et
Hét désigne un cycle hétérocyclique à 5 chaînons issu du groupe constitué par thiophène, thiazole, isothiazole, pyrrole et de manière particulièrement préférable pyrazole, pouvant porter 1-2 substituants R¹ identiques ou différents,
où la chaîne -(CH₂)ₙ- peut éventuellement être également interrompue par un chaînon -CH(CH₃)- ou -C(CH₃)₂-, et les sels des acides carboxyliques (R³ = H).

2. Composé de formule I selon la revendication 1, choisi parmi le groupe suivant :
le 2-(6-(4-difluorométhoxyphénoxy)hexyl)oxirane-2-carboxylate d'éthyle
le 2-(5-(4-difluorométhoxyphénoxy)pentyl)oxirane-2-carboxylate d'éthyle
le 2-(5-(4-acétylphénoxy)pentyl)oxirane-2-carboxylate d'éthyle.

3. Médicament de formule I selon la revendication 1 ou 2.

4. Médicament selon la revendication 3, pour une utilisation dans le traitement et la prophylaxie de maladies à base de troubles du métabolisme du glucose et/ou des matières grasses, telles que les conditions à tolérance pathologique au glucose, le prédiabète, le diabète du type 2, les conditions présentant une insulino-résistance, les conditions présentant une cétogenèse pathologiquement augmentée, l'hyperlipidémie, l'artériosclérose et/ou la maladie coronarienne.

5. Préparation pharmaceutique, **caractérisée en ce qu'**elle comprend un ou plusieurs composés de formule I selon la revendication 1 ou 2, éventuellement conjointement avec des excipients et/ou des adjuvants.

6. Utilisation d'acides oxiranecarboxyliques arylalkyl- ou aryloxyalkyl-substitués selon la revendication 1 ou 2, pour la préparation de médicaments pour le traitement et la prophylaxie de maladies à base de troubles du métabolisme du glucose et/ou des matières grasses, telles que les conditions à tolérance pathologique au glucose, le prédiabète, le diabète du type 2, les conditions présentant une insuline-résistance, les conditions présentant une cétogenèse pathologiquement augmentée, l'hyperlipidémie, l'artériosclérose et/ou la maladie coronarienne.
